# FASCICULE DE BREVET EUROPEEN

(11) **EP 0 608 648 B1**
(45) Date de publication et mention de la délivrance du brevet: **21.05.1997**
(21) Numéro de dépôt: 93403121.2
(22) Date de dépôt: 21.12.1993
(51) Int. Cl.: A61K 35/72

(54) **Composition contenant une très forte quantité de vitamines A pour une dose**
Hochdosierte Vitamine A-Zusammensetzungen
High dose vitamin A compositions

(30) Priorité: 28.01.1993 FR 9300861
(43) Date de publication de la demande: 03.08.1994
(73) Titulaire: Grimberg, Georges Serge, F-75007 Paris (FR)
(72) Inventeur: Grimberg, Georges Serge, F-75007 Paris (FR)
(74) Mandataire: Madeuf, René Louis

(56) Documents cités:
- FR-A- 2 431 863
- EUROPEAN JOURNAL OF HAEMATOLOGY, vol.40, no.5, Mai 1988 pages 460 - 465 LIE S.O. ET AL 'HIGH-DOSE RETINOL IN CHILDREN WITH ACUTE MYELOGENOUS LEUKAEMIA IN REMISSION'
- THE JOURNAL OF CANCER RESEARCH AND CLINICAL ONCOLOGY, vol.116, no.5, 1990 pages 503 - 506 BOCCARDO F. ET AL 'PHASE II STUDY OF TAMOXIFEN AND HIGH-DOSE RETINYL ACETATE IN PATIENTS WITH ADVANCED BREAST CANCER'

## Description

On connaît déjà des compositions thérapeutiques formées d'associations de vitamines A et de différents principes actifs ayant une activité thérapeutique en vue de pallier les désordres survenant dans la sphère ORL.

Le médicament décrit dans le brevet FR-A-2 228 470 contient de la vitamine A, du soufre, de la cystine et de la levure. Sa prescription est de 900.000 UI de vitamine A pour quinze jours. Cette thérapeutique préconisée quinze à vingt jours par mois pendant trois mois fait absorber au patient une quantité de vitamine A de 2.700.000 UI au moins.

Pour renforcer l'immunité contre une rhinite, cela fait apparaître un risque qui est très largement supérieur au bénéfice, et surtout pour des femmes en période de grossesse. Trois mois de traitement sont actuellement inconcevables.

De même le FR-A-2 647 784 décrit une composition thérapeutique nouvelle de la vitamine A mais à dose physiologique et elle ne répond pas aux conditions nécessaires à un traitement d'une rhinite aiguë ou grippale.

En effet, en thérapeutique, le problème posé aujourd'hui est dénommé BENEFICE-RISQUE et devient la base même de tout médicament.

Dans le cas de rhinite aiguë ou grippale, il est apparu qu'il faut frapper fort et vite pendant une période très courte pour que le BENEFICE-RISQUE soit positif.

Dans ce cas, les réactions secondaires sont pratiquement nulles et c'est le cas en particulier pour les femmes sous contraceptif oral.

La présente invention contient une forte dose de vitamine A qui est deux à trois fois la dose du médicament décrit dans le FR-A-2 228 470 et vingt a trente fois supérieure au médicament décrit dans le FR-A-2 647 784 et la durée du traitement est de trois à quatre jours d'ou il s'ensuit qu'il n'y a aucun risque et qu'une rhinite guérit en trois ou quatre jours.

On connaît d'autres compositions thérapeutiques contenant également de la vitamine A et on peut citer par exemple la composition selon FR-A-2 431 863.

Cependant, une dose de cette composition contient une proportion relativement faible de vitamine A et est associée à un complexe vitaminique B car cette composition est utilisable en dermatologie comme le montre les différents exemples cités dans le document.

L'utilisation de vitamine A est également connue dans le traitement de la leucémie et on peut citer a cet effet la revue "European Journal of Haemathology" qui, au volume 40 No 5 de Mai 1988, pages 460-465 montre l'utilisation de vitamine A associée à de la cytarabine (chimiothérapie). De même, l'utilisation de vitamine A est connue dans le traitement du cancer du sein et on peut citer à ce sujet la revue "The Journal of Cancer Research and Clinical Oncology" qui, au volume 116 No 5 de 1990, pages 503-506 montre l'utilisation de vitamine A associée au tamoxifen (anti-oestrogène utilisé en hormonothérapie) pour le traitement du cancer du sein à l'état avancé.

Les caractéristiques de l'invention sont ainsi données aux revendications annexées.

La nouvelle composition thérapeutique dans laquelle le soufre organique (L. Cystine base) est additionné au soufre sublimé lavé et qui contient de la levure morte qui est le plus souvent de la levure type saccharomycèse, a donné les résultats suivants :
Premier groupe : Vingt malades ayant une rhinite aiguë ont été traités avec trois doses du médicament 40.000 U.I. par jour pendant quatre jours.
Deuxième groupe : Vingt malades ayant une rhinite aiguë ont été traités avec trois doses pendant quatre jours correspondant à la composition thérapeutique décrite dans le brevet FR-A-2 228 470.
Troisième groupe : Vingt malades ayant une rhinite aiguë ont été traités avec trois doses pendant quatre jours correspondant à la composition thérapeutique décrite dans le brevet No 2 647 784.

Les vingt malades du premier groupe traités avec la formule du nouveau médicament sont les seuls à avoir été guéris en quatre jours.

On a conclu, en conséquence, que les essais cliniques ont prouvé le bien fondé de la nouvelle formule qui agit de manière forte et vite pendant un temps très court, devenant ainsi la panacée de la rhinite aiguë et donc de l'immunostimulation d'une manière générale pour une réponse rapide.

## Revendications

1. Composition thérapeutique nouvelle remédiant aux désordres survenant dans la sphère ORL et permettant un traitement durant trois ou quatre jours, caractérisée en ce qu'une dose contient :
| | |
|---|---|
| Vitamine A : | 40 000 UI à 60 000 UI |
| L.Cystine base | 217,8 mg |
| Soufre sublimé lavé | 66 mg |
| Levure morte | 232,2 mg |

2. Composition thérapeutique nouvelle suivant la revendication 1, caractérisée en ce que la levure morte est de la levure type saccharomycèse.

## Claims

1. New therapeutic composition remedying the disorders appearing in the otolaryngological sphere and enabling a treatment lasting three to four days, characterized in that one dose contains :
| | |
|---|---|
| Vitamin A | 40,000 to 60,000 IU |
| L. Cystin Base | 217.8 mg |
| Washed sublimed sulphur | 66 mg |
| Dead yeast | 232.2 mg |

2. New therapeutic composition according to claim 1, characterized in that the dead yeast is a saccharomyces type yeast.

## Patentansprüche

1. Neue, therapeutische Zusammensetzung, welche auf dem Gebiet der Hals-Nasen-Ohrenheilkunde auftretenden Störungen abhilft und eine drei- oder viertägige Behandlung ermöglicht, **dadurch gekennzeichnet**, daß sie eine Dosis
| | |
|---|---|
| 40 000 IE bis 60 000 IE | Vitamin A |
| 217,8 mg | Basis-L. Cystine (L. Cystine base) |
| 66 mg | gewaschene Schwefelblume |
| 232,2 mg | totes Gärungsmittel (Levure morte) |
enthält.

2. Neue, therapeutische Zusammensetzung nach Anspruch 1, dadurch gekennzeichnet, daß das tote Gärungsmittel eine Hefe der Gattung Saccharomyces ist.
